# EUROPEAN PATENT SPECIFICATION

(11) **EP 0 820 304 B1**
(45) Date of publication and mention of the grant of the patent: **21.06.2000**
(21) Application number: 96912001.3
(22) Date of filing: 01.04.1996
(51) Int. Cl.: A61K 39/118, A61K 39/39

(54) **CHLAMYDIA VACCINES**
CHLAMYDIA IMPFSTOFFEN
VACCINS CONTRE LES INFECTIONS A CHLAMYDIA

(30) Priority: 03.04.1995 GB 9506863
(43) Date of publication of application: 28.01.1998
(73) Proprietor: SMITHKLINE BEECHAM BIOLOGICALS S.A., 1330 Rixensart (BE)
(72) Inventor: PRIEELS, Jean-Paul, 1330 Rixensart (BE); SLAOUI, Moncef Mohamed, 1330 Rixensart (BE); MAISONNEUVE, Jean-François, Uni. des Sciences, 5032 Isnes (BE); VERLANT, Vincent, Uni. des Sciences, 5032 Isnes (BE)
(74) Representative: Dalton, Marcus Jonathan William
(86) International application number: EP9601463
(87) International publication number: WO9631236

(56) References cited:
- WO-A-93/01832
- WO-A-94/00153
- THE JOURNAL OF INFECTION, vol. 25, no. SUPPLEMENT 1, July 1992, pages 11-26, XP002011474 MICHAEL E. WARD: "CHLAMYDIAL VACCINES-FUTURE TRENDS"

## Description

The present invention relates to a vaccine formulation for the prevention of Chlamydia infections. In particular, to a formulation containing recombinant MOMP adjuvants with QS21 and 3D-MPL.

The obligate intracellular bacteria *Chlamydia trachomatis* infects mucosal epithelial cells of the conjunctiva and of the urogenital tract, causing a wide spectrum of human diseases such as trachoma and genital infections which can result in long term sequelae. Trachoma, which is endemic in several developing countries, is the world's leading cause of preventable blindness; genital infections, which represent around 3 million cases per year in the US, rend annually 200, 000 women infertile following Chlamydia salpingitis (1). Therefore, this pathogen is a significant public health problem and efforts are made to set up a vaccine against human Chlamydia infections.

Vaccine trials performed in man and non-human primates using the whole organism as immunogen gave serovar-specific protection but some of the vaccinees developed more severe reactions upon reinfection (2). Several studies have demonstrated that the pathology associated with Chlamydia infection is immunologically mediated (3); moreover, a purified Chlamydia 57 kDa (Hsp60) was shown to elicit a pathology similar to reinfection in animals previously infected (4, 5). This observation led to the conclusion that protection against *Chlamydia trachomatis* could only be achieved using a subunit vaccine.

The *Chlamydia trachomatis* species is stereotyped into 15 serovars which are placed into 3 serogroups: the B complex (serovars B, Ba, D, E, L1 and L2), the intermediate complex (serovars F, G, K, L3) and the C complex (serovars A, C, H, I and J) (6). Sexually transmitted diseases (STD) are caused by serovars D to K which cover the 3 serogroups. Thus a subunit vaccine against Chlamydia STD should protect against multiple serovars that are more or less antigenically related.

For the design of a subunit vaccine, much interest has been focused on the serotyping antigen which consist in the 40 kDa major outer membrane protein (MOMP). This protein which was shown to function *in vitro* as a porin (7), is present during the whole life cycle of the bacteria (8); this principal surface protein is highly immunogenic in humans and animals. The MOMP display 4 variable domains (VD) surrounded by five constant regions that are highly conserved among serovars (9, 10). *In vitro* and *in vivo* neutralizing B-cell epitopes have been mapped on VDs (11, 12, 13, 14, 15) whereas T-cell epitopes have been identified in both variable and constant domains (16, 17). Recombinant MOMP has been expressed in *E. coli* by different authors (18, 19, 20); however, Manning *et al.* shown that their recombinant protein failed to react with a monoclonal antibody that recognize a conformational MOMP epitope (18).

Immunizations with recombinant or purified MOMP followed by homotypic or heterotypic *Chlamydia* challenge have been performed in different animal models with variable effects on the parameters of the infection (21, 22, 23). An elegant experimental model of salpingitis has been developed in mice in which intrauterine inoculation of a human strain of *Chlamydia trachomatis* leads to long term infertility (24, 25). In a heterotypic challenge experiment, Tuffrey *et al.* have shown that parenteral and mucosal immunization with rMOMP absorbed on alhydrogel reduced the severity of the salpingitis and the duration of the lower genital tract colonization, respectively. However, the preparation conferred no protection against infertility resulting from infection (23). Future trends of Chlamydial vaccines are discussed in Ward, M.E., Journal of Infection, 1992, 25, Supplement 1, 11-26.

Both cell mediated and humoral immunity seem to play a protective role in the genital pathologies caused by *Chlamydia trachomatis.* However, Rank's group suggests that in mice T-cell mediated immunity is the principal immune mechanism for controlling chlamydial genital disease (26, 27, 28) and CD4 and CD8 positive T-cells have been shown to contribute to anti-chlamydial immunity *in vivo* (29, 30).

3 De-O-acylated monophosphoryl lipid A is known from GB2 220 211B (Ribi). Chemically it is a mixture of 3-deacylated monophosphoryl lipid A with 4, 5 or 6 acylated chains and is manufactured by Ribi Immunochem Montana.

QS21 is a Hplc purified non toxic fraction of a saponin from the bark of the South American tree Quillaja saponaria molina and its method of its production is disclosed (as QA21) in US Patent No. 5,057,540. Vaccines comprising both QS21 and 3D-MPL are disclosed in International Patent Publication No. WO94/00153.

The present invention provides for the first time a vaccine composition which is effective in conferring protection against infertility resulting from Chlamydia infections.

Accordingly the present invention provides a vaccine formulation comprising 3D-MPL, QS21 and MOMP from Chlamydia. In particular, the vaccine contains MOMP from the B complex serogroup. Preferably the vaccine contains at least a MOMP from serovar L2, but may additionally contain antigens from other serovars, such as D and E.

In a preferred embodiment, QS21 is presented with a sterol since such compositions show decreased reactogenicity and improves the stability of QS21 to base - mediated hydrolysis.

In preferred compositions of the invention, the QS21 is associated with liposome structure containing cholesterol (hereinafter referred to as DQ). Such adjuvant compositions are described in copending UK Patent Applications 9508326.7 and 9513107.4 (published as WO 96/33739) whose disclosure is incorporated herein by reference. The antigen and the MPL are, in this preferred formulation, outside the structure of the liposome.

Vaccine preparation is generally described in New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of protein in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-1000 µg of protein, preferably 2-100 µg, most preferably 4-40 µg. An optimal amount for a particular vaccine can be ascertained by standard studies involving observation of appropriate immune responses in subjects. Following an initial vaccination, subjects may receive one or several booster immunisation adequately spaced.

The formulations of the present invention maybe used for both prophylactic and therapeutic purposes.

Accordingly in one aspect, the invention provides a method of treatment comprising administering an effective amount of a vaccine of the present invention to a patient.

In an embodiment of the invention the MOMP antigen is from Serovart 2 and is produced in E.Coli by means of recombinant DNA techniques. In such circumstances the protein is produced without its signal sequence.

In the present invention, adjuvantation of the antigen with or without MPL+QS21 strongly influenced the IgG1 : IgG2a ratio in immunized groups; immunization with MPL+QS21 was associated with low IgG1 : IgG2a ratios and partial protection while immunization without MPL+QS21 led to higher IgG1 : IgG2a ratio and gave no protection. Interestingly, the switching to IgG2a antibody production by B cell is mediated by gamma interferon which are produced by the Th1 subset of T-helper lymphocytes whereas IgG1 production is mediated by interleukin-4 secreted by Th2 cells (40). As IgG2a production is controlled by Th1 cell products, it would seem likely that the protected groups presented a MPL+QS21 driven Th1 cell activation. In human and in mouse models, Th1 cytokines, IL-2 and IFN-gamma, are generally associated with resistance to infection with intracellular pathogens whereas Th2 cytokines, IL-4 and IL-10, are associated with progressive disease. This can be illustrated with the resistance or susceptibility of inbred strains of mice to *Leishmania major* that correlates with the induction of specific Th1 or Th2 response (41). Moreover, interferon gamma has anti-chlamydial activity *in vitro* and is involved in resolving the infection *in vivo* (42, 43). Thus, immunostimulants driven Th1 cytokines could be responsible for the protection observed in this vaccination experiment.

Two other observations support the hypothesis of the installation of a protective cell-mediated immunity in the rMOMP+MPL+QS21 vaccinated groups: firstly, the absence of specific secretory IgA in the vaginal secretions and the non neutralizing nature of the strong seric antibody response rule out the possibility of having a humoral protection; secondly, the heterotypic character of the protection obtained using two distinct serovars differing considerably at the level of the MOMP VDs sequence suggests that T-cell epitopes from processed conserved domains of MOMP could be the agent of the protection against infertility.

The following illustrates the invention.

### 1. MATERIAL AND METHODS

### 1.1 Chlamydial strains and animals

*Chlamydia trachomatis* serovar F strain NIl isolated by Tuffrey *et al.* and kindly provided by Dr. J. Orfila and *Chlamydia trachomatis* serovar L2 strain 434 (ATCC, Rockville, Md) were used in this study. *Chlamydia* were inoculated on Mc Coy cells (ATCC, Rockville,Md) at a concentration of approximately 10⁶ inclusion forming units/ml (IFU/ml) in MEM supplemented with 10% foetal calf serum (FCS) (Gibco BRL). After 1h centrifugation (1500g) and 2h incubation at 37°C (5 % CO2), the inoculum was removed and cell were refed with fresh medium supplemented with 0.5 µg/ml cycloheximide (Sigma). After incubation at 37°C for 48h, cells were disrupted with glass beads, harvested in 250 mM sucrose, 10 mM sodium phosphate, 5mM L-glutamic acid pH 7.2 (SPG) to an approximate concentration of 10⁷ to 10⁸ IFU/ml and stored at -70°C.

Female C3H/HeOuJ (H-2^{k}) mice, 6-8 weeks old were obtained from Iffa Credo (France). 8 to 10 weeks old males from the same strain (B & K , U.K.) were used for breeding.

### 1.2 PCR amplification and plasmid constructions

*Amplification.* MOMP serovar L2 DNA was obtained by lysis of 10 µl of the chlamydial inoculum in 240 µl of lysis buffer and PCR amplification as described previously by Denamur *et al.* (33). Synthetic oligonucleotides 5'-GAGACTCCCAT**GGATCC**ACTGCCTGTGGGGAATCCTGC-3' and 5'-TTAGAAGCGGAATTGTGCATTTAC-3' (SB Biologicals, Belgium) were chosen from the published sequence (34). The 5' oligonucleotide contained the nucleotide sequence coding for the amino-terminus of the mature MOMP (underlined) preceded by a BamHI restriction endonuclease site (bold type). The amplification was carried out using Pfu DNA polymerase (Stratagen) and a Koch Light NBS Thermal Cycler (New Brunswick). A right sized PCR product was purified from a 1 % agarose gel using a Geneclean II kit (Bio 101).

### 1.3 Cloning

The amplified serovar L2 MOMP DNA was rendered blunt-end with the Klenow fragment of DNA polymerase I, ligated into pGEM4Z (Promega) previously digested with SmaI and transformed into *E. coli* JM109 using the standard CaCL₂ protocole. Restriction analysis was performed on the resulting clones and a right DNA construct was amplified and purified using a nucleobond PC-100 kit (Macherey-Nagel). MOMP DNA was then excised from pGEM4Z-MOMP by digestion with BamHI and inserted into BamHI digested pET15 (Novagen) downstream the T7*lac* promotor and the His.Tag sequence. Right clones were selected by restriction analysis after transformation into the *E. coli* strain DH10B (stratagen); the complete nucleotide sequence of the cloned DNA was verified by the dideoxy chain termination method (35). A pET15-MOMP plasmid preparation was finally used to transform the BL21(DE3) strain (Novagen) which is able to promote the recombinant product expression as it possess an IPTG inducible T7 polymerase.

### 1.4 Immunogen production, characterization, purification and formulation

1.4.1 *Production and characterization.* pET15-MOMP transformed BL21(DE3) bacteria were cultured into LB medium (Gibco BRL) supplemented with 200 µg/ml ampicilline (Sigma). Expression was induced by adding 1mM IPTG when the culture optical density measured at 600 nm has reached 0.6 to 0.8. Cells are harvested 3h after induction, washed 3 times with PBS and lysed in sample buffer containing 2% SDS and 5 % mercaptoethanol. Samples were heated for 3 min at 95°C and total proteins were separated by 12% SDS-PAGE using molecular weight markers separated on the same gel (Gibco BRL). For immunoblotting, the 12% SD-PAGE separated proteins were transferred onto nitrocellulose and detected using mAbs L2 I-45 and L2 I-10 kindly provided by Dr. H. Caldwell and a goat anti-MOMP antiserum (Chemicon). The cellular location of the rMOMP was determined by cell fractionation as described in Maniatis *et al.*(36); pellet and supernatants were resuspended or adjusted in sample buffer and analysed like the total cell lysate.

1.4.2 *Purification.* A 100 ml volume of 3h IPTG induced culture was lysed with lysozyme and deoxycholate as described previously by Marston *et al.* (37). The cell lysate was centifugated (12 000g for 15 min.), the pellet was resuspended in 2 ml of SDS PAGE sample buffer containing 2 % SDS but no mercaptoethanol and boiled for 3 min. The lysate was centrifugated (12 000 g for 15 min.), the pellet was discarded and the supernatant adjusted to 20 mM Tris-HCl pH 7.9, 0.5% SDS, 500 mM NaCl, 5mM imidazole in final concentration. The sample was then loaded onto a chromatography column containing 2 ml His.Bind resin (Novagen); the ion metal affinity chromatography was then achieved according to the manufacturer's procedure. Identity and purity of the eluted product was estimated by SDS-PAGE under reducing conditions followed by Coomassie blue staining or immunoblotting (see above). Protein concentration was determined by the Lowry's assay. rMOMP containing fractions were pooled and dialysed overnight against PBS using Slide A Lyser cassettes (Pierce).

*Formulation of the antigen.* Three formulations were tested:
1) MOMP+QS21 3DMPL
2) MOMP+SB62 Oil in water emulsion
3) MOMP, SB62, QS21 3DMPL

This was carried out according to the procedure described in WO 95/17210 and/or WO94/00153. Briefly purified and dialysed rMOMP was diluted in PBS to 25 µg/ml (200 µl) for injection with 5 µg MPL + 10 µg QS21 or to 50 µg/ml (100 µl) for mixing with 100 µl oil in water emulsion referred to as SB62 with or without 5 µg MPL/10 µg QS21. For each of the formulations the vaccines were prepared as follow:
1) MPL/QS21 formulated by adding MPL (as 100 nm particles) to MOMP antigen, followed by buffer and then QS21.
2) MPL/QS21/SB62 formulated by adding antigen to buffer followed by SB62 followed by MPL as 100 nm particle followed by QS21. In this formulation it is believed that the antigen is out (ie outside the emulsion droplet), the MPL is out and most of the QS21 is out.
3) SB62 formulated by adding SB62 to antigen in buffer. Antigen is out.

### 1.5 Vaccination in the mice model of salpingitis, fertility and serological follow-up

Groups of ten female C3H/HeOuJ were subcutaneously immunized at the basis of the tail with 2 x 5 µg rMOMP in 200 µl of the different formulations at weeks 0 and 2; the control group was sham-immunized following the same schedule with the emulsion containing 5 µg MPL and 10 µg QS21. Inoculation was carried out at week 6 following the protocole described previously by Tuffrey *et al.* (24). Briefly, mice were given 2.5 mg progesterone subcutaneously (Depo-Provera, Upjohn) 7 days before challenge which was performed by bilateral intrauterine inoculation with 5 10⁵ IFU *Chlamydia trachomatis* NI1 in 100 µl SPG or 100 µl of a Mc Coy cell extract.

At week 10, treated mice were caged with male for 3 months for fertility assessment (1 male for 2 females, with weekly rotation of the male within each group). The parameters that were calculated over the breeding period were the mean number of newborn mice per group (M) and the average litter size (N).

Blood was taken at weeks 6 and the sera were analysed for rMOMP-specific antibodies, serovar F strain NI1 chlamydial inclusions recognition and neutralization of heterologus (NI1) *in vitro* infection. Vaginal washes were collected at week 6 by pipeting 50 µl of PBS into and out of the vagina several times and analysed for rMOMP specific secretory IgA antibodies.

### 1.6 Serological analysis

1.6.1 *Determination of anti-rMOMP antibodies.* The rMOMP-specific IgG or IgA titers were determined using the rMOMP as antigen in an enzyme-linked immunosorbent assay (ELISA). The plates (Maxisorp, Nunc) were coated overnight at 4°C with a 5 µg/ml solution of antigen in 10 mM carbonate/bicarbonate buffer, pH 9.6 buffer, washed with 0.1 % Tween 20 PBS (washing buffer) and blocked for 1h at 37 °C with PBS 3% BSA (Sigma). Test sera were serially diluted in washing buffer containing 0.5 % BSA (incubation buffer) for 1h at 37 °C. The plates were washed and incubated for 1h at 37°C with a horseradish peroxydase-congugated goat anti-murine IgG or IgA (Sigma) . After washing, the substrate orthophenylen-diamine (Sigma) was added at room temperature for 20 min; the reaction was stopped by addition of 2M H₂SO₄ and the absorbance at 492 nm was measured on a Labsystems Multiskan. The anti-rMOMP IgG or IgA titer was expressed as the reciprocal of the serum sample dilution giving a midpoint absorbance value.

For each serum sample, the rMOMP-specific IgG response was dissected into rMOMP-specific IgG2a, IgG2b and IgG1 ratios in a direct ELISA as described above with some modifications. Test sera were incubated in triplicate, the plates were washed and biotinylated goat anti-murine IgG2a, IgG2b or IgG1 (Amersham) diluted in incubation buffer were added to each lane of the triplicate. After 1h at 37°C, the plates were washed and incubated for 1h with a streptavidine horseradish peroxydase complex (Amersham). Revelation and titer determination were carried out as described above. The prevalence of each of the 3 IgG subtypes expressed in percent was calculated as the ratio between this IgG subtype titer and the total of the titers determined for the 3 subtypes.

1.6.2 *Heterotypic detection of chlamydial inclusions.* Mac Coy cells were cultured in sterile flat-bottom 96-well microplates (Nunc) and confluent monolayers were infected with approximately 5 10⁴ IFU of *Chlamydia trachomatis* serovar F strain NI1. 24h post-infection, the cells were washed with PBS and fixed 10 min with methanol. Washing was repeated and 100 µl of the serum samples diluted 1/100 with PBS were incubated for 1h at 37°C. The plates were washed and treated with horseradish peroxydase-conjugated goat anti-mouse IgG (Sigma) for 1h at 37°C. After washing with PBS, the antibody binding was visualized by addition of diaminobenzidine tetrahydrochloride (DAB, Sigma). The presence of anti-rMOMP IgG reveled NI1 inclusions was assessed using an inverted optical microscope.

1.6.3 *Heterotypic in vitro neutralizing activity.* The complement independent *in vitro* neutralization assay was performed as described by Su *et al.* (38) with some modifications. Briefly 50 µl twofold SPG dilution of decomplemented individual mouse sera were added to 10⁵ IFU of serovar F strain NI1 diluted in 50 µl SPG. The mix was incubated 30 min at 37°C (5% CO2) and then the 100 µl were inoculated onto HBSS (Gibco BRL) washed Syrian Hamster Kidney cells (HaK, ATCC, Rockville,Md) and incubated for 2h at 37°C (5% CO2). Then, the inocula were removed, the cells were washed with HBSS and MEM containing 10% FCS, 50 µg/ml gentamycin and 0.5 µg/ml cycloheximide was added. After 24 h incubation at 37 °C, cells were fixed and inclusions were immunochemically detected as described above using a commercial goat anti-MOMP antisera (Chemicon) and an alkaline phosphatase conjugated rabbit anti-goat (Sigma). 5-brom-4-chloro-3-indolyl phosphate/nitro blue tetrazolium (BCIP/NBT, Sigma) was used as the substrate for the enzymatic reaction. IFU were quantitated by counting 5 fields at a magnification of 200x using an inverted microscope. The mean IFU number per field obtained with the sample sera was expressed as percentage reduction of the mean IFU number obtained with negative control mixture which contained serum from naive mice. The neutralization titers (NT 50) were calculated as the reciprocal of the serum sample dilution giving 50% reduction of the infectivity.

### 1.7 Results

*1.7.1 Recombinant antigen expression and characterization*

A PCR amplified DNA fragment containing the nucleotide sequence coding for mature MOMP serovar L2 was inserted in the right reading frame and orientation into the pET15 expression vector; the nucleotide sequence of the chlamydial protein and the fusion joint with the polynucleotide stretch encoding the 5'-terminal His-Tag peptide were as predicted by the design of the cloning strategy. After cell fractionation, the expression product was located in the insoluble fraction of *E. coli* which suggests that it was expressed in the form of insoluble inclusion bodies. Recombinant MOMP containing pellet was solubilized in 2% SDS buffer and run onto a ion metal affinity chromatography column in which immobilized nickel ions were used to chelate histidine residues beared by the His.tag peptide fused with the recombinant MOMP. The purified protein which has been washed and eluted in buffers devoid of SDS displayed the predicted molecular weight and was immunoreactive with anti-MOMP monoclonal and polyclonal antibodies as shown by SDS-PAGE and Western blot analysis respectively. After dialysis, the rMOMP concentration was situated between 500 µg/ml and 1 mg/ml and purity of the recombinant product was estimated at 90%.

*1.7.2 Effect of immunization with adjuvanted rMOMP on mouse serological response and fertility after heterotypic challenge*

The results of the experiment designed to evaluate the prophylactic potential of differently adjuvanted recombinant MOMP are presented as outlined in table 1. Groups 4 and 5 were subcutaneously immunized with rMOMP adjuvanted with 5 µg MPL and 10 µg QS21; in group 4, the adjuvanted recombinant protein was prepared in the SB62 emulsion containing squalene and alpha tocopherol as the oil phase and Tween 80 as the surfactant. Group 6 was subcutaneously immunized with rMOMP combined with the same SB62 emulsion as group 4 but without immunostimulants. Three control groups were also designed. A group of non treated animals (group 1), a group of sham-immunized mice using both immunostimulant combined with SB62 emulsion (group 2) and group 3 which was immunized like group 4 but was devoted to mock-infection, group 2, 4, 5 and 6 were challenged with the heterotypic *Chlamydia trachomatis* strain NI1.

*1.7.3 Effect of immunization on the serological response.* In order to evaluate the immunogenicity of the different preparation, IgG titers were measured by ELISA on sera drawn 4 weeks after the second dose of vaccine (day of the challenge) and the arithmetic mean titers (AMT) were calculated for each group. Immunisation with two injections of 5 µg of rMOMP led to the appearance of high level of anti-rMOMP IgG . As shown in table 1, AMTs were virtually the same for groups 4 and 6 but combination of emusion and immunostimulants resulted in a twofold increase of the rMOMP specific IgG mean titer. Animals sham-immunized with adjuvants only (group 2) had no significant antibody titers against the chlamydial recombinant antigen. In any groups, no specific rMOMP secretory IgA were detected in the vaginal washes collected just before challenge.

As shown in table 1, a significant difference in the IgG subclasses profile was observed between the immunostimulants containing groups (4 and 5) and group 6 which was immunized with SB62 emulsified rMOMP. The utilization of MPL+QS21 was shown to significantly enhance the relative level of IgG2a and decrease the relative level of IgG1 ; the maximum effect of this phenomenon was reached with the non-emulsified preparation.

To ascertain whether the rMOMP specific IgG were able to cross-react with Chlamydia of the heterotypic infecting strain, sera were diluted 1:100 and individually tested in an immuno-enzymatic assay for their ability to recognize chlamydial inclusion on methanol fixed infected cells. All the mouse sera from each immunized group were shown to contain IgG reacting with the *Chlamydia trachomatis* serovar F strain NI1 utilized for the challenge. Therefore, they were all tested for *in vitro* complement-independent neutralizing activity against this strain using sera from sham-immunized mice as negative controls . Results were inconsistent in comparison to those obtained by ELISA and immunoenzymatic-assay since none of the immune sera was able to significantly reduce the chlamydial infectivity.

*1.7.4 Effect of heterotypic immunization on the fertility after challenge.* Eight weeks after the last immunization (or sham-immunization) with rMOMP and 4 weeks after intrauterine infection (or mock-infection), mice were mated with male for 2 months. The outcome of the challenge with the heterologus strain NI1 on C3H/He0uJ mice fertility was measured through the following parameters : the mean number of newborn per group (N) and the average litter size (M). Compared to untreated mice, fertility of Chlamydia inoculated mice in group 2 (sham-immunized) were significantly altered; this result confirmed the validity of the animal model in this particular case. Animals immunized and mock-infected (group 3) presented reduced parameters of fertility compared to untreated ones; this group designed to take non-pathological alteration of the animal fertility into consideration was used as control to evaluate the prophylactic potential of the rMOMP formulations. As shown in table 1 significant differences in the fertility levels were observed between the immunostimulants co-vaccinated groups (groups 4 and 5) and group 6 which was immunized with SB62 emulsified rMOMP. Group 5 displayed the best results with 7 out to 10 mice giving birth to litters and maximal fertility parameters; the N value reached 50% of the value attributed to the control group 3 and the M value was comparable to those calculated for the same control group. On the contrary, the fertility parameters displayed by group 6 lacking immunostimulants is comparable to those obtained in the infection control group 2. rMOMP immunization combining immunostimulants and the SB62 emulsion also resulted in protection against infertility but the utilization of the SB62 emulsion seemed to partially decrease the protection rates. Thus, the utilization of MPL plus QS21 was shown to offer a partial protection against upper genital tract chlamydial infection and the maximum effect of this phenomenon was reached with the non-emulsified preparation.

### 1.8 Conclusion

Our results show that parenteral immunization with MPL+QS21 adjuvanted rMOMP partially prevent infertility caused by an heterologus chlamydial infection of the mouse genital tract; on the contrary, injection of SB62 emulsified rMOMP without both immunostimulants do not induce any protection. On the other hand, all the preparations elicited strong and relatively homogeneous MOMP specific total IgG response in the sera of immunized animals; those antibodies were able to cross-react with methanol fixed chlamydial inclusions of the heterotypic infecting strain but unable to reduce the chlamydial infectivity *in vitro.* Just before challenge, rMOMP specific IgA were not detectable in the vaginal secretion which is consistent with the parenteral antigen administration method. Thus, a comparison of total specific IgG titers or neutralization titers with the outcome of the pathology for all immunized groups revealed no significant correlation for any of these comparison.

Results from the present investigation demonstrate that a recombinant MOMP combine with MPL+QS21 immunostimulants is capable of eliciting immune protection against infertility caused by *Chlamydia trachomatis.*

### 2. SECOND SERIES OF EXPERIMENTS VARIOUS MOMP ADJUVANT FORMULATIONS

### 2.1 Material and methods were tested

Chlamydial and mouse strains were identical to those utilised in the experiment described in example 1.

### 2.2 MOMP production and formulation

The production and the use of the DNA construct pET15-MOMP for antigen production are described in Example 1. The antigen purification protocol was modified in order to produce larger quantities of endotoxin-free antigen. Briefly, 10 ml His. Bind resin (Novagen) were washed with 25 volumes of 2% SDS, 6M urea in water before performing the purification step according to the manufacturer while 250 ml of induced lysate pelleted by centrifugation was washed with 4M urea, 2M NaCl followed by 2 % Zwittergen (Calbiochem) before solubilisation in Tris-HCl pH 7.9, 0.5% SDS, 500 mM NaCl, 5mM imidazole. The antigen was eluted in Tris-HCl pH 7.9, 100 mM imidazole, extensively dialysed against 5 mM Tris-HCl pH 7.4 and filtered onto a 0.22 µm sterilising filter until (Millipore). A limulus amaoebocyte lysate test (Coatest, Chromogenix) was then used to assess the LPS content.

### 2.3 Formulation

The antigen was formulated as described in example 1 except that the quantity of MPL per dose was adjusted to 10 pg; a group utilising modified QS21 as described in copending UK applications 9508326.7, 9513107.4 (published as WO 96/33739 and referred as DQ) was also tested and added to the vaccine at the rate of 10 µg a dose. In more detail the vaccine was formulated by adding antigen to buffer. MPL as 100 nm particles was then added. In separate tube, QS21 was mixed with small unilamellar liposomes composed of dioleoylphosphatidylcholine (DOPC) and cholesterol (DOPC:cholesterol = 4:1 w/w) so that the QS21 to cholesterol ratio is 1:5. (Under these conditions all the QS21 is incorporated into the liposomal membrane). The QS21/SUV mix (called DQ) is then added to the antigen/MPL mix. In this formulation the antigen is out, the MPL is out, the QS21 is in liposomes.

### 2.4 Vaccination in the mice model of salpingitis, fertility, immunological and histological follow-up.

Immunisation, experimental infection and sampling were scheduled as described above except that the negative control group was sham-immunised with the emulsion containing 10 µg MPL and 10 µg QS21 and that an extra group immunised with the antigen combined with MPL + DQ was added. Groups were composed of 15 mice: 10 of them were mated over a 8 week period while 5 were sacrificed 2 weeks post challenge for histopathological and immunocytochemical analysis. The parameters used for estimating group's fertility are: F (number of mice which littered one time or more divided by the total number of mice), M (number of newborn mice (dead or alive) divided by the number of litters) and N (number of newborn mice (dead or alive) divided by the total number of mice).

Sera and vaginal washes were analysed for rMOMP-specific antibodies by ELISA, sera were also examined for serovar F strain NI1 chlamydial inclusions recognition and neutralisation of heterologus (NI1) in vitro infection. All the techniques are described supra.

Upper half genital tract (ovary, oviduct and top of the uterine horn) were embedded in OCT compound (Tissue-TEK, Miles), snap frozen and frozen sections (10 µm) were mounted on glass slides (Superforst, Menzel-glaser). Sections were air-dried, fixed in acetone for 5 minutes and then stored at -70°C. For histopathological analysis, water rehydrated sections were stained with haematoxylin (H) and eosin (E). For immunocytochemical staining, sections were rehydrated in PBS, incubated for 60 minutes with 2 µg of biotinylated rat anti-mouse CD4 or CD8 mAb (Serotec) in 100 µl PBS, washed 2 times with PBS and reincubated 30 minutes with a 1/2000 dilution of HRP-strepatvidin (Zymed). After washing, colour was developed with a liquid DAB kit (Zymed), countersigned with haematoxylin and permanently mounted in acrytol (Surgipath).

### 2.5 Interferon gamma assay

Mice were subcutaneously injected into the basis of the tail with 200 µl of formulation on weeks 0 and 2; the control group was sham-immunised with 10 µg MPL and 10 µg QS21 combined to the emulsion. Animals were bled for serological analysis and then sacrificed on week 4, spleens were aseptically removed, pooled and single cell suspension were prepared for restimulation with 1 µg/ml rMOMP or with 4 /ml Con A (Boerhinger Mannheim) as a control. Therefore cultures were set up in flat bottom 24-well culture plates using 10⁶ responder cells per ml of RPMI 1640 with 10% foetal calf serum (Gibco-BRL). Supernatants harvested at 96 hours post restimulation were assayed for IFN-gamma using a commercial ELISA kit (Cytoscreen, Biosource).

### 2.6 Results

*2.6.1 Antigen*

After dialysis, the rMOMP concentration was estimated around 2 mg/ml and endotoxins contamination was below 0.05 EU/µg rMOMP/

*2.6.2* Effect of immunisation with adjuvanted rMOMP on the mice humoral immune response, on their fertility after heterotypic challenge and on the inflammatory infiltrate resulting from infection.

The results of the experiment designed to evaluate the prophylactic potential of differently adjuvanted rMOMP are presented as outlined in table 2.

*2.6.3* Effect of immunisation on the humoral response. Humoral response after vaccination was assessed in sera and vaginal washes collected on the day of the challenge. All the formulations of the antigen gave similar anti-rMOMP IgG geometric mean titers (GMT) of around 30,000. Animals sham-immunised with adjuvants only had no significant antibody titers against the chlamydial recombinant antigen. In any groups, no rMOMP-specific secretory IgA were detected in the vaginal washes collected just before challenge. Isotyping the rMOMP-specific IgG response revealed that the presence of MPL and QS21 or DQ enhanced the relative level of IgG2a when compared to the response evoked by MOMP in emulsion alone. All the immune sera were shown to contain IgG reacting with methanol-fixed inclusions of the serovar FC trachomatis strain NI1 utilised for the challenge.

*2.6.4* Effect of heterotypic immunisation on the fertility after challenge. The outcome of the challenge with the heterologus strain NI1 on mice fertility was measured through F, N and M parameters defined in the experimental procedures. For each group, values of those parameters calculated over the duration of the mating period are presented at table 1. In opposition to the sham-infected group, infection of the sham-immunised group led to nearly complete infertility, indicating that the observed infertility is induced by C.trachomatis and not by the manipulation of the animals. Among the groups immunised with rMOMP adjuvanted with both immunostimulants, MPL+DQ formulation of the antigen led to partial protection: in this group the values of the F and N parameters reach around 80% of those recorded in the sham-infected group (positive control). On the contrary, immunisation prior to challenge with rMOMP formulated in the emulsion led to low values of the F and N fertility parameters.

*2.6.5 Histopathological changes after challenge*

In the histopathological counterpart of the fertility experiment, classical HE coloration performed on tissue sections revealed no markedly difference in the inflammation scores of the oviducts and the ovaries between sham-immunised and vaccinated groups. However, when looking at the frequence of T-cell subsets by immunocytochemical straining, CD4 positive T-cells were only detected in the MPL+DQ vaccinated group (4 of 4 mice), whereas CD8 positive T-cells were detected in immunised as well sham-immunised groups (table 3). Thus, in this experiment, CD4 positive infiltrating T-cells were only found in the MPL+DQ vaccinated group which was the only group to be protected in the fertility counterpart of the experiment.

*2.6.6 Effect of the formulation on IFN-gamma secretion upon in vitro restimulation*

The cellular activation induced by the antigen formulations (table 4) was analysed in a separate experiment. On one hand, spleen cells isolated from animals vaccinated with rMOMP combined with MPL+QS21 or MPL+DQ and in vitro restimulated with the antigen displayed IFN-gamma concentrations in their culture supernatants which are comparable to those stimulated during the same period with 4 µg/ml of concanavaline A. On the other hand, cells isolated from animals sham-vaccinated or vaccinated with rMOMP devoid of immunostimulants did not produce detectable levels of IFN-gamma while their counterpart co-cultured with ConA were all positive for that cytokine. Serological analysis performed on pools of sera from each group revealed that IFN-gamma secretion was associated with an enhancement of the antigen-specific IgG2a ratio.

### CONCLUSION

Put together, data from the challenge trial and the IFN-gamma detection assay suggest that, in mouse, the combination of the two adjuvants MPL and QS21 or DQ with a recombinant MOMP induces an antigen-specific Th1-like immune response determined by IFN-gamma secretion and elevated IgG2a ratios, which can result in protection against infertility resulting from chlamydial infection.

### REFERENCES

1. Washington AE, Johnson RE and Sanders LL. *Chlamydia trachomatis* infections in the United States: what are they costing us. JAMA 1987, 257, 2070-2072.
2. Grayston JT and Wang SP. New knowledge of *Chlamydiae* and the diseases they cause. The Journal of Infectious Diseases 1975, 132: 87-105.
3. Grayston JT, Wang SP, Yeh LJ, and Kuo CC. Importance of reinfection in the pathogenesis of trachoma. Reviews of Infectious Diseases 1985, 7, 717-725
4 Morrison RP, RJ Belland, K Lyng and HD Caldwell. Chalmydial disease pathogenesis. The 57-kD Chlamydial hypersensitivity antigen is a stress response protein. J. Exp. Med. 1989, 170, 1271-1283
5. Blander SJ and Amortegui AJ. Mice immunized with a chlamydial extract have no increase in early protective immunity despite increased inflammation following genital infection by the mouse pneumonitis agent of *Chlamydia trachomatis.* Infec. Immun. 1994, 62, 3617-3624. µg
6. Wang SP, Kuo CC, Barnes RC, Stephens RS and Grayston JT. Immunotyping of *Chlamydia trachomatis* with monoclonal antibodies. The Journal of Infectious Diseases 1985, 152, 791-800.
7. Bavoil P, Ohlin A, and Schachter J. Role of disulfide bonding in outer membrane structure and permeability in *Chlamydia trachomatis.* Infect. Immun. 1984, 44, 479-485.
8. Hatch TP, Miceli M, Sublett JE. Synthesis of disulfide-bonded outer membrane proteins during development cycle of *Chlamydia psittaci* and *Chlamydia trachomatis.* J. Bacteriol. 1986, 165, 379-385.
9. Stephens RS, R Sanchez-Pescador, EA Wagar, C Inouye and MS Urdea. Diversity of *Chlamydia trachomatis* Major Outer Membrane Protein genes. J. Bacteriol. 1987, 169, 3879-3885.
10. Yuan Y, Zhang YX, Watkins NG, and Caldwell HD. Nucleotide and deduced amino acid sequences for the four variable domains of the major outer mebrane proteins of the 15 *Chlamydia trachomatis* serovars. Infect. Immun. 1989, 57, 1040-1049.
11. Baehr W, Zhang YX, Joseph T, Su H, Nano FE, Everett EK and Calwell HD. Mapping antigenic domains expressed by *Chlamydia trachomatis* major outer membrane protein genes. Proc. Natl Acad. Sci. USA 1988, 85, 4000-4004
12. Lucero ME and Kuo CC. Neutralization of *Chlamydia trachomatis* cell culture infection by serovar-specific monoclonal antibodies. Infect. Immun. 1985, 50, 595-597
13. Zhang YX, Stewart S, Joseph T, Taylor HR and HD Caldwell. Protective monoclonal antibodies recognize epitopes located on the major outer membrane protein of *Chlamydia trachomatis.* J. Imunol. 1987, 138, 575-581.
14. Peterson E, Zhong G, Carlson E and de la Maza LM. Protective role of magnesium in the neutralization by monoclonal antibodies of *Chlamydia trachomatis* infectivity. Infect. Immun. 1988, 56, 885-891.
15. Zhang YX, Stewart SJ and Caldwell HD. Protective monoclonal antibodies to *Chlamydia trachomatis* serovar- and serogroup- specific major outer membrane protein determinants. Infect. Immun. 1989, 57, 636-638
16. Allen JE, RM Loksley and RS Stephens. A single peptide from the major outer membrane protein of *Chlamydia trachomatis* elicits T cell help for the production of antibodies to protective determinants. J. Immunol. 1991, 147, 674-679
17. Su H, RP Morrison, NG Watkins and HD Caldwell. Identification and characterization of T helper cell epitopes of the major outer membrane protein of *Chlamydia trachomatis.* J. Exp. Med. 1990, 172, 203-212
18. Manning DS and SJ Stewart. Expression of the major outer membrane protein of Chalmydia trachomatis in *Escherichia coli.* Infect. Immun. 1993, 61, 4093-4098.
19. Koehler JE, Birkelund S and Stephens RS. Overexpression and surface localization of the *Chlamydia trachomatis* major outer membrane protein in *Escherichia coli.* Molecular Microbiology 1992, 6, 1087-1094
20. Pickett MA, ME Ward and IN Clarke. High level expression and epitope localization of the major outer membrane protein of *Chlamydia trachomatis* serovar L1. Molecular Microbiology 1988, 2, 681-685.
21. Taylor HR, J Whittum-Hudson, J Schachter, HD Caldwell and RA Prendergast. Oral immunization with chlamydial major outer membrane protein (MOMP). Investigative Ophthalmology and visual Science 1988, 29, 1847-1853
22. Batteiger BE, RG Rank, PM Bavoil and LSF Soderberg. Partial protection against genital reinfection by immunization of guinea-pig with isolated mouter membrane proteins of the chlamydial agent of guinea-pig inclusion conjunctivitis. Journal of General Microbiology 1993, 139, 2965-2972.
23. Tuffrey M, F Alexander, W Conlan, C Woods and M Ward, Heterotypic protection of mice against chlamydial salpingitis and colonization of the lower genital tract with a human serovar F isolate of *Chlamydia trachomatis* by prior immunization with recombinant L1 major outer-membrane protein. Journal of General Microbiology 1992, 138, 1707-1715.
24. Tuffrey M, P Falder, J Gale and D Taylor -Robinson. Salpingitis in mice induced by human strains of *Chlamydia trachomatis.* Br. J. exp. Path. 1986, 67, 605-616.
25. Tuffrey M, P Falder, J Gale, R Quinn and D Taylor -Robinson.Infertility in mice infected genitally with a human strain of *Chlamydia trachomatis* . Br. J. exp. Path. 1986, 78, 251-260
26. Ramsey KH, LSF Soderberg and RG Rank. Resolution of Chlamydial genital infection in B-cell deficient mice and immunity to reinfection. Infect. Immun. 1988, 56, 1320-1325.
27. Rank RG, LSF Soderberg and AL Barron. Chronic chlamydial genital infection in congenitally athymic nude mice. Infect. Immun. 1985, 48, 847-849
28. Igietseme JU and RG Rank. Susceptibility to reinfection after a primary chlamydial genital infection is associated with a decrease of antigen-specific T cell in the genital tract. Infect. Immun. 1991, 59, 1346-1351
29. Igietseme JU, KH Ramsey, DM Magee, DM Williams TJ Kincy and RG Rank. Resolution of murine chlamydial genital infection by the adoptive transfer of a biovar-specific, TH1 Lymphocyte clone. Regional Immunology 1993, 5, 317-324.
30. Igietseme JU, DM Magee, DM Williams and RG Rank. Role for CD8+ T cell in antichlamydial immunity defined by chlamydial-specific T-lymphocyte clones. Infect. Immun. 1994, 62 , 5195-5197.
31. Myers KR and JT Ulrich. Effective use of monophosphoryl lipid A as an adjuvant. *In* Novel vaccine strategies. Mucosal, adjuvant and genetic appoaches. International Business Communication, Ma, USA.
32. Newman MJ, JY Wu, BH Gardner, KJ Munroe, D Leombruno, J Recchia, CR Kensil and RT Coughlin. Saponin adjuvant induction of ovalbumin-specific CD8+ cytotoxic T lymphocyte responses. The Journal of Immunology 1992, 148, 2357-2362.
33. Denamur E, C Sayada, A Souriau, J Orfila, A Rodolakis and J Elion. Restriction pattern of the major outer membrane protein gene provides evidence for a homogeneous invasive group among ruminant isolates of *Chlamydia psittaci.* Journal of General Microbiology 1991, 137, 2525-2530.
34. Zhang YX, SG Morrison and HD Caldwell. The nucleotide sequence of major outer membrane gene of *Chlamydia trachomatis* serovar F. Nucleic Acids Research 1990, 18, 1061.
35. Tabor S and CC Richardson. Proc. Natl Acad Sci USA 1989, 86, 4076-4080.
36. Sambrook J, EF Fritsch and T Maniatis. *Molecular cloning. A Laboratory Manual.* Second edition. Cold Spring Harbor Laboratory Press, 1989.
37. Marston FAO. The purification of eukaryotic polypeptides expressed in *Escherichia coli.* In: *DNA cloning: A Practical approach* (ed. DM Glover) vol 3 p. 59. IRL Press, Oxford.
38. Su H and HD Caldwell. Immunogenicity of a synthetic oligopeptide corresponding to antigenically common T-helper and B-cell neutralizing epitopes of the major outer membrane protein of *Chlamydia trachomatis.* Vaccine 1993, 11, 1159-11166.
39. Tuffrey M, F Alexander, C Inman and ME Ward. Correlaton of infertility with altered tubal morphology and function in mice with salpingitis induced by a human genital-tract isolate of *Chlamydia trachomatis.* J. Reprod. Fert. 1990, 88, 295-305.
40. Snapper CM and WE Paul. Interferon-gamma and B cell stimulatory factor-1 reciprocally regulate Ig isotype production. Science 1987, 236, 944-947.
41. Heinzel FP, MD Sadick, SS Mutha and RM Loksley. Production of Interferon-gamma, Interleukin 2, Interleukin 4, and Interleukin 10 by CD4+ lymphocytes *in vivo* during healing and progressive murine leishmaniasis. Proc. Natl Acad. Sci. USA 1991, 88, 7011.
42. Byrne Gi, LK Lehmann and GJ landry. Induction of tryptophan catabolism for gamma interferon mediated inhibition of intracellular *Chlamydia psittaci* replication in T24 cells. Infect, Immun. 1986, 53: 347.
43. Rank RG, KH Ramsey, EA Pack and DM Williams. Effect of gamma interferon resolution of murine chlamydial genital infection. Infect. Immun. 1992. 60, 4427-4429.

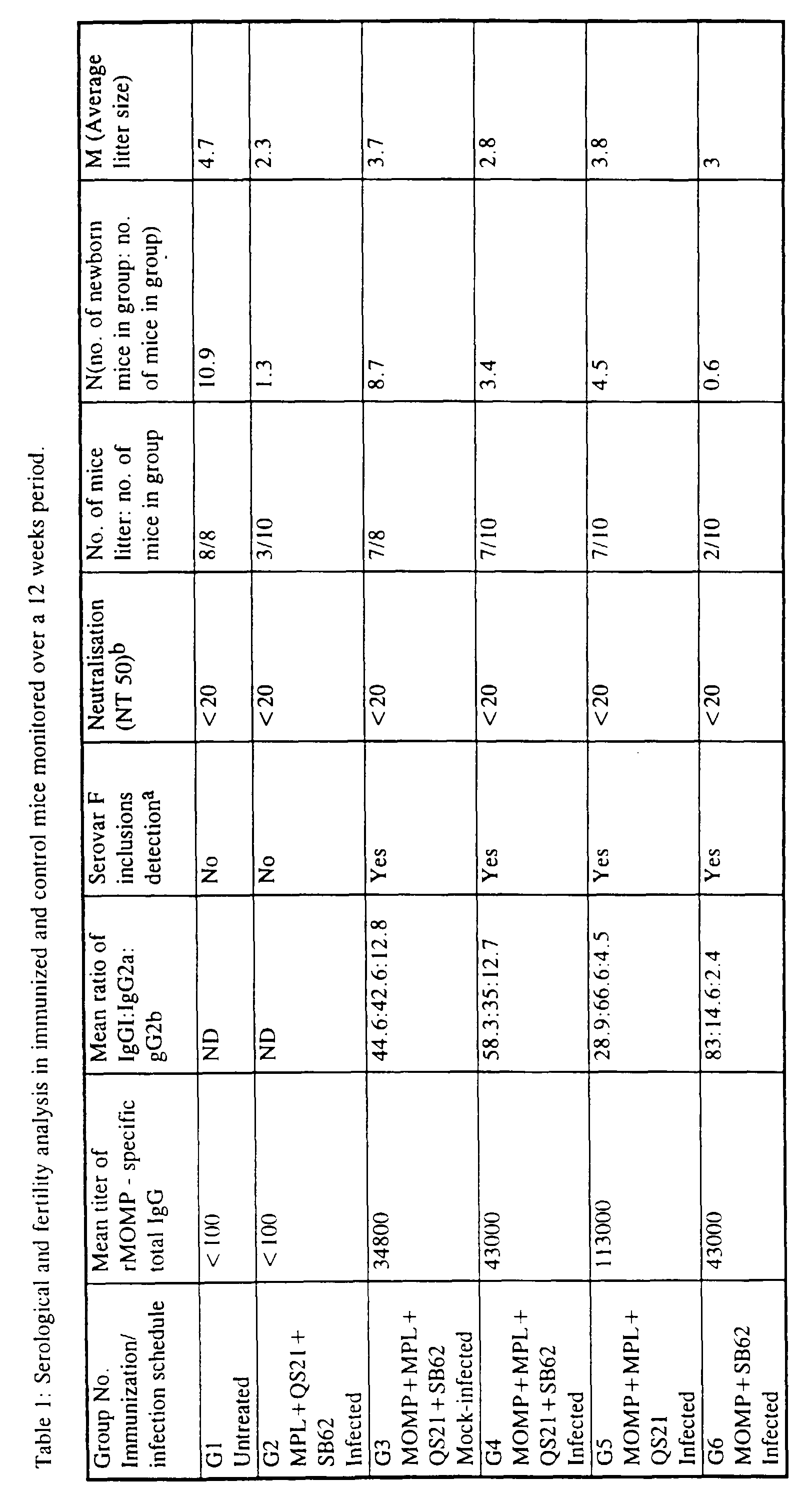

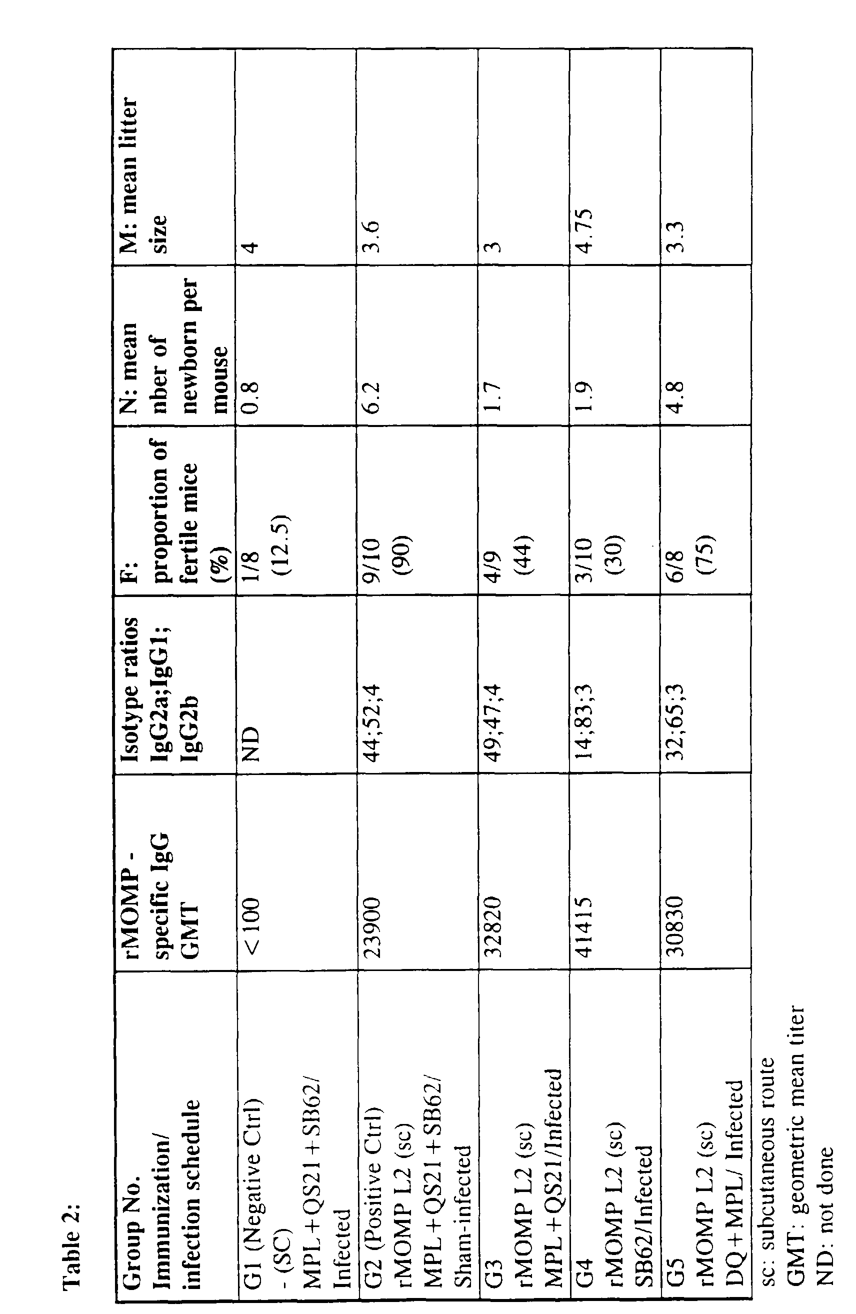

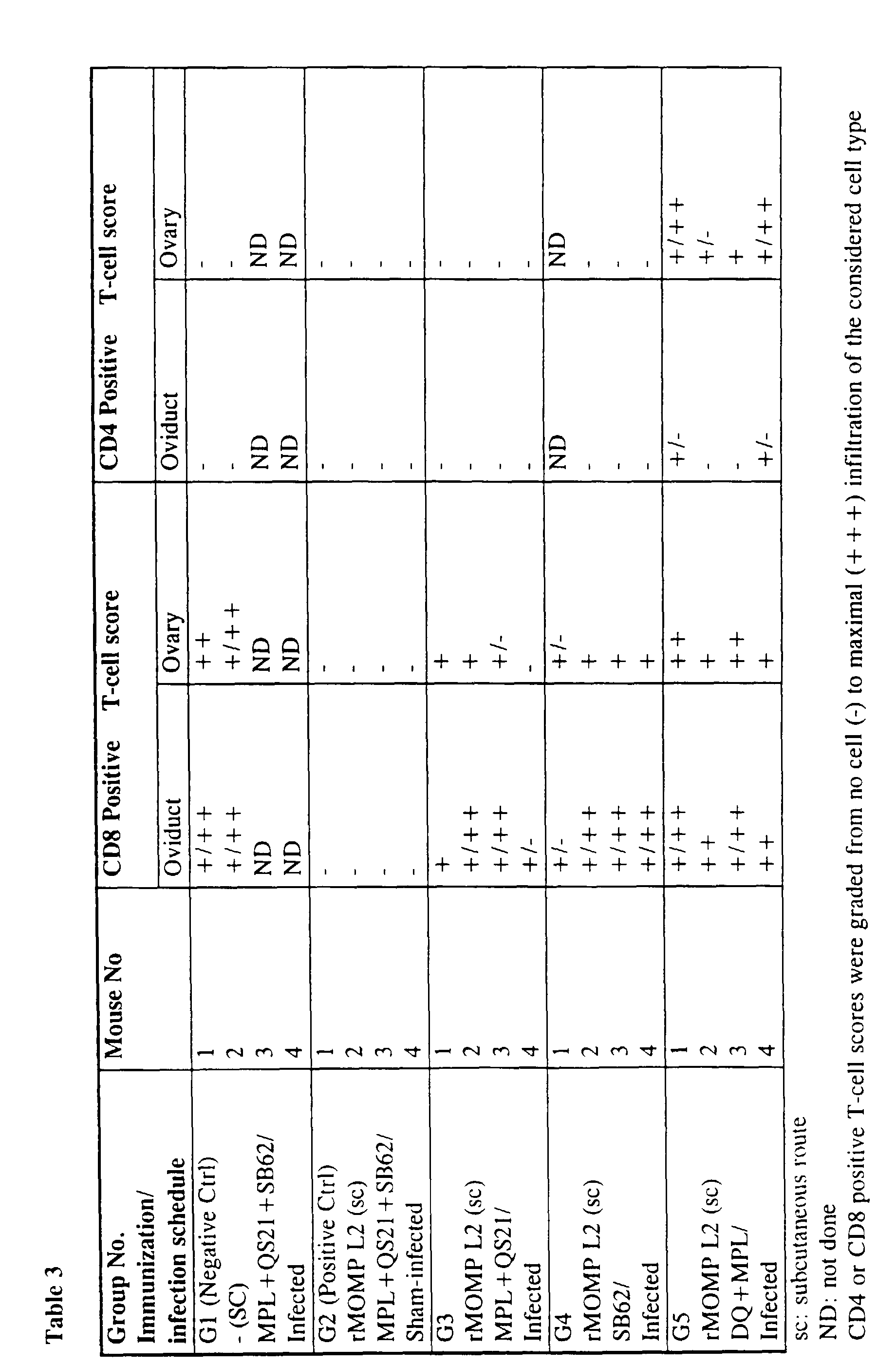

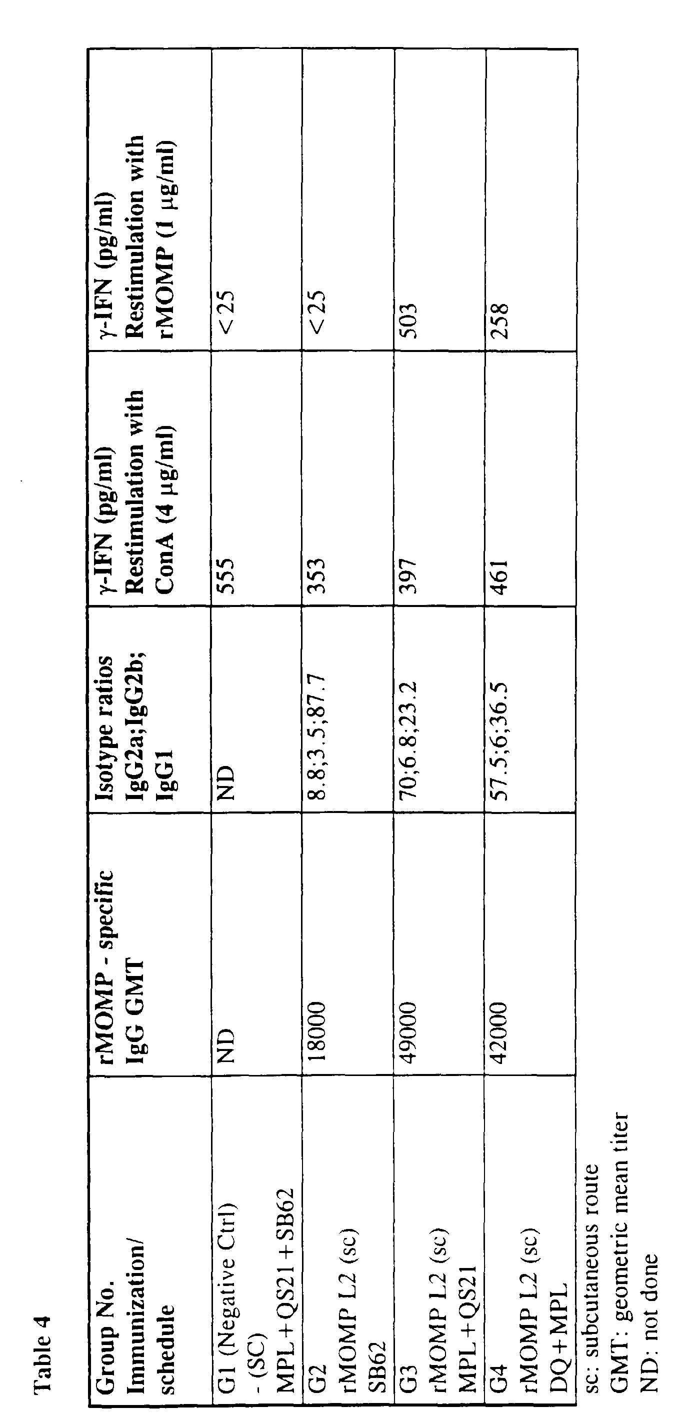

## Claims

1. A vaccine composition comprising a MOMP protein from Chlamydia in conjunction with QS21 and 3DMPL.

2. A vaccine as claimed in claim 1 where the protein is derived from a Chlamydia L2 Serovar.

3. A vaccine as claimed in claim 1 or 2 additionally comprising a protein from a different serovar.

4. A vaccine as claimed in claim 1 which additionally comprises a sterol.

5. A vaccine as claimed in claim 4 wherein the sterol is cholesterol.

6. A vaccine as claimed in claim 5 wherein QS21 is associated with a cholesterol containing liposome.

7. A vaccine as claimed in claim 6 wherein the antigen is out of the liposome.

8. A vaccine as claimed in any of claims 5 to 7 wherein the ratio of QS21 to cholesterol is about 1:5.

9. A method of manufacturing a vaccine comprising adding 3D-MPL to MOMP antigen, and then adding QS21.

10. A vaccine as claimed herein for use as a medicament.

11. Use of MOMP protein from Chlamydia in conjunction with QS21 and 3D-MPL in the manufacture of a vaccine to reduce Chlamydia induced infertility.

## Patentansprüche

1. Impfstoffzusammensetzung, umfassend ein MOMP-Protein von Chlamydia in Verbindung mit QS21 und 3DMPL.

2. Impfstoff nach Anspruch 1, wobei das Protein von einem Chlamydia-L2-Serovar stammt.

3. Impfstoff nach Anspruch 1 oder 2, der zusätzlich ein Protein von einem unterschiedlichen Serovar umfasst.

4. Impfstoff nach Anspruch 1, der zusätzlich ein Sterin umfasst.

5. Impfstoff nach Anspruch 4, wobei das Sterin Cholesterin ist.

6. Impfstoff nach Anspruch 5, wobei QS21 mit einem Cholesterin enthaltenden Liposom assoziiert ist.

7. Impfstoff nach Anspruch 6, wobei das Antigen außerhalb des Liposoms ist.

8. Impfstoff nach einem der Ansprüche 5 bis 7, wobei das Verhältnis von QS21 zu Cholesterin etwa 1:5 beträgt.

9. Verfahren zur Herstellung eines Impfstoffes, umfassend die Zugabe vom 3D-MPL zu dem MOMP-Antigen und die anschließende Zugabe von QS21.

10. Impfstoff wie hier beansprucht zur Verwendung als ein Medikament.

11. Verwendung eines MOMP-Proteins von Chlamydia in Verbindung mit QS21 und 3D-MPL für die Herstellung eines Impfstoffes zur Reduktion von Chlamydia-induzierter Infertilität.

## Revendications

1. Composition vaccinale comprenant une protéine MOMP provenant de Chlamydia en association avec QS21 et 3D-MPL.

2. Vaccin suivant la revendication 1, dans lequel la protéine dérive d'un sérovar L2 de Chlamydia.

3. Vaccin suivant les revendications 1 ou 2, comprenant de plus une protéine provenant d'un sérovar différent.

4. Vaccin suivant la revendication 1, qui comprend de plus un stérol.

5. Vaccin suivant la revendication 4, dans lequel le stérol est le cholestérol.

6. Vaccin suivant la revendication 5, dans lequel QS21 est associé avec un liposome contenant du cholestérol.

7. Vaccin suivant la revendication 6, dans lequel l'antigène est à l'extérieur du liposome.

8. Vaccin suivant l'une quelconque des revendication 5 à 7, dans lequel le rapport de QS21 au cholestérol est d'environ 1:5.

9. Procédé de fabrication d'un vaccin comprenant l'addition de 3D-MPL à un antigène MOMP, et ensuite l'addition de QS21.

10. Vaccin suivant les revendications précédentes utile en tant que médicament.

11. Utilisation de protéine MOMP provenant de Chlamydia en association avec QS21 et 3D-MPL dans la fabrication d'un vaccin pour réduire une infertilité induite par Chlamydia.
